# EUROPEAN PATENT APPLICATION

(11) **EP 3 329 940 A1**
(43) Date of publication of application: **06.06.2018**
(21) Application number: 16830793.2
(22) Date of filing: 25.07.2016
(51) Int. Cl.: A61K 47/34, A61K 9/00, A61K 38/13

(54) **NON-IRRITANT OPHTHALMIC COMPOSITION CONTAINING CYCLOSPORIN, AND CONVENIENT PREPARATION METHOD**

(30) Priority: 29.07.2015 KR 20150107237
(71) Applicant: Kukje Pharm. Ind. Co., Ltd, Seongnam-si, Gyeonggi-do 13517 (KR)
(72) Inventor: LEE, Dongwoo, Seoul 05298 (KR); BAE, Hyunju, Suwon-si Gyeonggi-do 16322 (KR); KIM, Younggwan, Ansan-si Gyeonggi-do 15463 (KR)
(74) Representative: Louis Pöhlau Lohrentz
(86) International application number: PCT/KR2016/008091
(87) International publication number: WO 2017/018760

(57) **Abstract**

Provided is an aqueous-medium ophthalmic composition containing: cyclosporin as an active ingredient; and a polyoxyethylene stearate as a solubilizing agent.

## Description

### Technical Field

The present invention relates to an ophthalmic composition comprising cyclosporin.

### Background Art

Dry eye syndrome has a very high prevalence rate in old people, and recently, the prevalence rate in the youth generation is increasing due to the increase in the hours of using smartphones and computers.

Since therapies for the dry eye syndrome are very limited, only cyclosporin, diquafosol sodium and hyaluronic acids have been currently known as drugs having a therapeutic activity for the dry eye syndrome. In particular, hyaluronic acid is close to an adjuvant therapeutic agent, not a therapeutic agent. Among these drugs, it has been known that cyclosporin can be used widely for mild to severe dry eye syndrome, and it is highly safe.

However, it has been known that since cyclosporin has a low solubility to water (solubility: 0.004% w/w), it is hard to prepare cyclosporin as a ophthalmic composition.

In order to solve such problem, the technology for preparing an emulsion using castor oil as a solubilizing agent and polysorbate 80 as an emulsifying agent has been developed (Korean Patent Nos. 368181 and 450703). However, this technology should use a high-pressure homogenizer or a high-speed shear machine, which makes the equipment complicated, increases the manufacturing cost, and raises the temperature when emulsifying. In particular, this technology should use a high-pressure steam sterilization method because it uses a polymer such as carbomer in order to stabilize the composition, and thus there is a problem that this technology cannot use heat-sensitive ingredients.

In order to solve such problems, technologies for preparing a nano emulsion using polyethoxylated castor oil such as polyoxyl 35 castor oil (Cremophor EP) and a surfactant such as propylene glycol dicaprylocaprate (Korean Patent No. 1151235), or preparing a nano emulsion using polyethoxylated castor oil and ethanol as an auxiliary solvent (Korean Patent No. 1211902) have been developed.

These technologies all use castor oil or polyoxyl 35 castor oil in order to dissolve the poorly water-soluble cyclosporine. Here, it has been reported that such oils or surfactants exhibit cytotoxicity to conjunctival cells or toxicity to endothelial cells or epidermal cells (Investigative Ophthalmology & Visual Science, Nov 2007, vol 48, No. 11; European Journal of Pharmaceutical science 45 (2012) 492-498; J. Pharmacol Exp Ther 1977 Apr; 201(1): 259-266; Agent Actions 12, 64-80, 1982; etc.).

In this regard, researches have been conducted on technologies for preparing a cyclosporin emulsion without using castor oil or polyethoxylated castor oil, and accordingly a technology for preparing a cyclosporin ophthalmic composition by using propylene glycol, polysorbate 80 and purified water treated with high-frequency (Korean Patent No. 1510764) has been developed.

However, in the above technology, a shearing force should be applied while continuously stirring strongly at a rate of 3000-5000 rpm during and for 5-10 minutes after adding a mixture solution of propylene glycol wherein cyclosporin is dissolved and a surfactant into purified water treated with high frequency, which makes the equipment complicated, and increases the manufacturing cost.

### Detailed description of the invention

### Technical task

The technical task of the present invention is to easily prepare an ophthalmic composition by dissolving and emulsifying the poorly water-soluble cyclosporin without using castor oil or polyethoxylated castor oil which is harmful to the human body, and to improve the safety of the medicine.

### Means for achieving the technical task

As a means for solving the technical task, the present invention discloses an ophthalmic composition comprising: cyclosporin as an active ingredient; and a polyoxyethylene stearate (preferably, polyoxyl 40 stearate) as a solubilizing agent.

### Effect of the invention

According to the present invention, cyclosporin is solubilized by using ingredients harmless to the human body, and thus the composition is very safe. In particular, by using only substances with excellent effect in solubilizing cyclosporin or stabilizing the cyclosporine-containing ophthalmic composition among the substances whose safety is guaranteed, the solubility, stability and safety of cyclosporin may be improved, and the irritation may be reduced when applied to the eyes. Also, at the time of preparing the ophthalmic composition according to the present invention, processes such as heating or high-frequency treating, etc. are not required, and the ophthalmic composition of a nano emulsion having a cyclosporin particle size of 50 nm or below may be prepared by simply stirring the surfactant and the aqueous medium (at a stirring rate of 500 to 700 rpm or below) without using a high-pressure homogenizer or a high-speed shear machine. Therefore, the manufacturing equipment does not need to be complicated and the costs may be reduced. Also, the ophthalmic composition according to the present invention does not have to use ethanol as an auxiliary solvent, can be sterilized by filtering, has excellent stability, and reduced irritation.

### Brief description of the drawings

Fig. 1 illustrates the result of measuring a distribution of the particle size of the ophthalmic composition (example 4-C) prepared according to the present invention in experimental example 1; and
Fig. 2 illustrates the result of measuring a distribution of the particle size of the control group in experimental example 1.

### Best mode for carrying out the invention

The present invention relates to an ophthalmic composition comprising: cyclosporin as an active ingredient; and a polyoxyethylene stearate (preferably, polyoxyl 40 stearate (Myrj 52)) as a solubilizing agent.

In the present invention, the content of cyclosporin is 0.01 to 1 % by weight, preferably 0.05 to 0.1 % by weight with respect to the total weight of the ophthalmic composition. In the present invention, the content of polyoxyethylene stearate is 3.0 to 7.0 % by weight, preferably 4.0 to 5.0 % by weight with respect to the total weight of the ophthalmic composition.

The ophthalmic composition of the present invention may further comprise a poloxamer as a surfactant. Preferably, the content of the poloxamer is 0.01 to 0.04 % by weight with respect to the total weight of the ophthalmic composition.

The ophthalmic composition of the present invention may further comprise a thickener. At least one of hydroxypropyl methyl cellulose, hydroxypropyl cellulose, hydroxyethyl cellulose, methyl cellulose, polyvinylpyrrolidone, polyvinyl alcohol, polyethylene may be selected as the thickener. Preferably, polyvinylpyrrolidone (povidone) is used as the thickener. Preferably, the thickener is used in a range of 0.01 to 1.8 % by weight with respect to the total weight of the composition.

The ophthalmic composition of the present invention may further comprise propylene glycol, etc. as a stabilizer. Preferably, propylene glycol is used in a range of 0.5 to 1.5 % by weight with respect to the total weight of the composition.

The ophthalmic composition of the present invention may further comprise a buffer. Citric acid, sodium citrate, edetate sodium, or aminocaproic acid, etc. may be used as the buffer.

The ophthalmic composition of the present invention may use sodium chloride, glycerin, etc. as an isotonic agent.

The ophthalmic composition according to the present invention is a nano emulsion of cyclosporin, and the average particle size of cyclosporin is about 50 nm or less (see experimental example 1).

The ophthalmic composition of the present invention is prepared by a method comprising the steps of: (a) dissolving cyclosporin in polyoxyl 40 stearate to prepare a first solution; (b) dissolving poloxamer 407, propylene glycol, a thickener, an isotonic agent, a buffer, etc. in purified water to prepare a second solution; and (c) adding the second solution to the first solution and simply stirring the mixture. In step (c) of the preparation method of the present invention, a homogenizer or a high-speed shear machine is not used at the time of or after adding the second solution to the first solution, and the nano emulsion can be prepared by simply stirring at a rate of 500-700 rpm or below.

### Mode for carrying out the invention

The present invention can improve the solubility, stability and safety of cyclosporin, and reduce irritation when applied to the eyes by using substances with excellent effect in solubilizing cyclosporin or stabilizing the cyclosporine-containing ophthalmic composition among the substances whose safety is guaranteed, and excluding ingredients whose side effects are known (e.g., castor oil, modified castor oil, Cremophor, phosphate, etc.).

Hereinafter, the present invention will be described in detail with reference to the following examples. The examples provided below are only for exemplifying the present invention, and are not intended to limit the scope of the invention.

### [Examples]

### <Example 1> Preparation of nano emulsion ophthalmic composition

According to the composition as in Table 1 below, cyclosporine is dissolved in polyoxyl 40 stearate, which is a solubilizing agent. The other excipients (i.e., poloxamer 407, polyvinylpyrrolidone, propylene glycol, citric acid, and sodium citrate) are dissolved in purified water. The purified water solution in which the excipients are dissolved is added to the polyoxyl 40 stearate in which the cyclosporine is dissolved and the mixture is stirred to prepare a nano emulsion. Then, purified water is added so that the total weight of the composition does not exceed 100 g. Then, NaOH or HCl aqueous solution is added to adjust the pH to range between 7.2 and 7.6, and purified water is added again so that the total weight reaches 100 g.

**[Table 1]**

| Ingredients | Example 1-A | Example 1-B |
|---|---|---|
| cyclosporine | 0.05g | 0.05g |
| polyoxyl 40 stearate | 5g | 4g |
| poloxamer 407 | 0.04g | 0.04g |
| propylene glycol | 1.0g | 1.0g |
| Povidone K90 | 0.5g | 0.5g |
| citric acid | 0.016g | 0.016g |
| sodium citrate | 1.26g | 1.26g |
| purified water | q.s. (so that the total weight could be 100g) | q.s. (so that the total weight could be 100g) |

### <Example 2> Nano emulsion composition per type of buffer

As shown in Table 2 below, the type, amount and ratio of the buffer were adjusted to prepare a nano emulsion composition with a similar method to example 1.

**[Table 2]**

| Ingredients | 2-A | 2-B | 2-C | 2-D | 2-E |
|---|---|---|---|---|---|
| cyclosporine | 0.05g | 0.05g | 0.05g | 0.05g | 0.05g |
| polyoxyl 40 stearate | 5g | 5g | 5g | 5g | 5g |
| poloxamer 407 | 0.04g | 0.04g | 0.04g | 0.04g | 0.04g |
| propylene glycol | 1.0g | 1.0g | 1.0g | 1.0g | 1.0g |
| Povidone K90 | 1.14g | 1.14g | 1.14g | 1.14g | 1.14g |
| citric acid | 0.016 | 0.01g | | | |
| sodium citrate | 1.26 | 0.9g | | | |
| edetate sodium | | | 0.05g | 0.02g | |
| amino caproic acid | | | | | 0.02g |
| purified water | q.s. | q.s. | q.s. | q.s. | q.s. |

### <Example 3> Nano emulsion composition per type of thickener

As shown in Table 3 below, the type, amount and ratio of thickener were adjusted to prepare a nano emulsion composition with a similar method to example 1.

**[Table 3]**

| Ingredients | 3-A | 3-B | 3-C | 3-D | 3-E | 3-F | 3-G |
|---|---|---|---|---|---|---|---|
| cyclosporine | 0.05g | 0.05g | 0.05g | 0.05g | 0.05g | 0.05g | 0.05g |
| polyoxyl 40 stearate | 5g | 5g | 5g | 5g | 5g | 5g | 5g |
| propylene glycol | 1.0g | 1.0g | 1.0g | 1.0g | 1.0g | 1.0g | 1.0g |
| poloxamer 407 | 0.04g | 0.04g | 0.04g | 0.04g | 0.04g | 0.04g | 0.04g |
| Povidone K90 | 1.14g | | | | | | |
| Povidone K25 | | 1.14g | | | | | |
| hydroxypropyl methylcellulose | | | 1.14g | | | | |
| hydroxypropyl cellulose | | | | 1.14g | | | |
| hydroxyethyl cellulose | | | | | 1.14g | | |
| methyl cellulose | | | | | | 1.14g | |
| polyvinyl alcohol | | | | | | | 1.14g |
| citric acid | 0.016g | 0.016g | 0.016g | 0.016g | 0.016g | 0.016g | 0.016g |
| sodium citrate | 1.26g | 1.26g | 1.26g | 1.26g | 1.26g | 1.26g | 1.26g |
| purified water | q.s. | q.s. | q.s. | q.s. | q.s. | q.s. | q.s. |

### <Example 4> Nano emulsion composition per concentration of cyclosporine

As shown in Table 4 below, the amount of cyclosporine and solubilizing agents were adjusted to prepare a nano emulsion composition with a similar method to example 1.

**[Table 4]**

| Ingredients | 4-A | 4-B | 4-C | 4-D | 4-E | 4-F | 4-G |
|---|---|---|---|---|---|---|---|
| cyclosporine | 0.05g | 0.05g | 0.05g | 0.1g | 0.1g | 0.1g | 0.1g |
| polyoxyl 40 stearate | 5g | 5g | 5g | 6g | 6g | 7g | 7g |
| Poloxamer 407 | 0.01g | 0.02g | 0.04g | 0.02g | 0.04g | 0.02g | 0.04g |
| propylene glycol | 1.0g | 1.0g | 1.0g | 1.0g | 1.0g | 1.0g | 1.0g |
| Povidone K90 | 1.14g | 1.14g | 1.14g | 1.14g | 1.14g | 1.14g | 1.14g |
| citric acid | 0.016g | 0.016g | 0.016g | 0.016g | 0.016g | 0.016g | 0.016g |
| sodium citrate | 1.26g | 1.26g | 1.26g | 1.26g | 1.26g | 1.26g | 1.26g |
| purified water | q.s. | q.s. | q.s. | q.s. | q.s. | q.s. | q.s. |

### [Experimental examples]

### <Experimental example 1> Measurement of particle size of nano emulsion

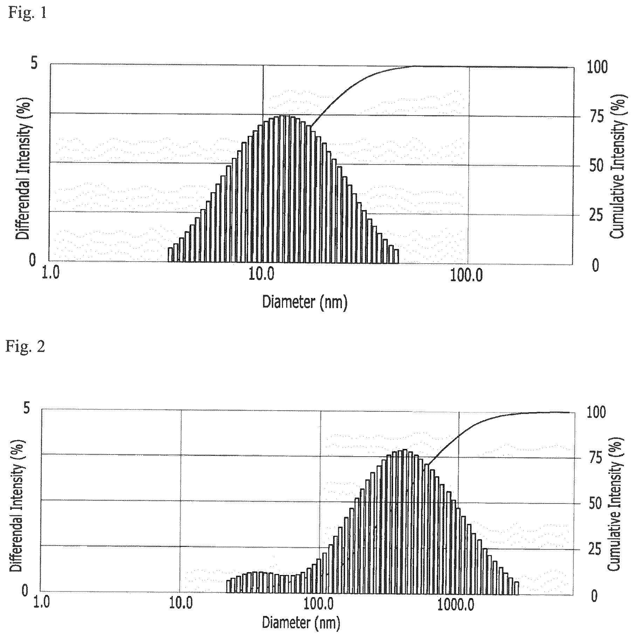

The particle size distribution and average particle size of the nano emulsion prepared in examples 1 to 4 above were measured without dilution by using ELSZ-1000 (Otsuka, Germany). A result of the measurement is as shown in Table 5 below and figs. 1 and 2:

**[Table 5]**

| Average particle size | | | | |
|---|---|---|---|---|
| | 1-B | 4-A | 4-C | Market product |
| average particle size (nm) | 13.2 | 14.8 | 14.5 | 335.3 |

When referring to Fig. 1 which illustrates the particle size of the composition in example 4-A, the particle size is 14.8 nm which is very small, and the particle size distribution is uniformly presented. In comparison, when measuring the average particle size of the product which is currently sold in the market, the particle size thereof is 335.8 nm which is large, and the particle size distribution is also very broad as illustrated in Fig. 2.

As such, since the particle size of cyclosporine in the ophthalmic composition of the present invention is small, the composition of the present invention has excellent stability, has a low sense of foreign matter and a low level of irritation when being administered, and is capable of being sterilized by filtering.

### <Experimental example 2> Stability testing

The composition prepared in the example was kept in an acceleration condition (40±2°C, relative humidity (RH) 25%) for 6 months to test the pH and content. The content was analyzed by using the HPLC under the following conditions:
HPLC analysis conditions: 210 nm wavelength, C8 column
Mobile phase: tetrahydrofuran 0.03M phosphoric acid solution (40:60)
Velocity of flow: 1 ml/min.

A result thereof is as shown in Table 6 below:

**[Table 6]**

| | | 1-B | 2-B | 4-A | 4-C |
|---|---|---|---|---|---|
| pH | initial | 7.17 | 7.19 | 7.19 | 7.38 |
| | after 6 months | 7.06 | 7.10 | 7.00 | 7.31 |
| content (%) | initial | 103.8 | 100.7 | 100.7 | 101.5 |
| | after 6 months | 104.5 | 101.1 | 101.1 | 100.2 |
| average particle size (nm) | initial | 13.2 | 13.4 | 14.8 | 14.5 |
| | after 6 months | 11.2 | 12.9 | 11.6 | 13.8 |
| properties | initial | Colorless. Transparent. No precipitation found. | Colorless. Transparent. No precipitation found. | Colorless. Transparent. No precipitation found. | Colorless. Transparent. No precipitation found |
| | after 6 months | Colorless. Transparent. No precipitation found. | Colorless. Transparent. No precipitation found. | Colorless. Transparent. No precipitation found. | Colorless. Transparent. No precipitation found. |

### <Experimental example 3>

With the same composition as in Table 7 below, a nano emulsion was prepared by using the similar method to example 1, and stability testing was conducted under the acceleration conditions in experimental example 2:

**[Table 7]**

| Function | Ingredients | 7-A | 7-B |
|---|---|---|---|
| active ingredient | cyclosporine | 0.05g | 0.05g |
| solubilizing agent | polyoxyl 40 stearate | 4g | 5g |
| thickener | Povidone K90 | 1.14g | 1.14g |
| stabilizer | propylene glycol | 1g | 1g |
| surfactant | poloxamer 407 | 0.04g | 0.04g |
| buffer | citric acid | 0.01g | 0.01g |
| buffer | sodium citrate | 0.9g | 0.9g |
| initial | pH | 7.28 | 7.25 |
| | content (%) | 102.5 | 101.0 |
| | osmotic pressure (mOsm) | 298 | 310 |
| after 2 months of acceleration | pH | 7.30 | 7.20 |
| | content (%) | 101.0 | 100.5 |
| after 4 months of acceleration | pH | 7.25 | 7.21 |
| | content (%) | 101.5 | 100.8 |
| after 6 months of acceleration | pH | 7.15 | 7.10 |
| | content (%) | 102.0 | 100.7 |
| | osmotic pressure | 308 | 318 |
| | (mOsm) | | |

From the above results, it may be noticed that the composition of the present invention shows excellent stability in the acceleration testing condition of a semipermeable container prescribed by Pharmaceutical Affairs Law.

As a control group, by varying the solubilizing agent, stabilizer, surfactant, etc., the ophthalmic composition (comparative examples 8-B, 8-C and 8-D; each total weight is 100g) prepared by stirring at a high speed of 3000-5000 rpm in purified water in the composition as in Table 8 below showed a sharp decrease in content, and precipitation was found in the test after 30 days of acceleration.

**[Table 8]**

| Ingredients | | Example 8-A | Example 8-B | Example 8-C | Example 8-D |
|---|---|---|---|---|---|
| active ingredients | cyclosporine | 0.05g | 0.05g | 0.05g | 0.05g |
| solubilizing agent | polyoxyl 40 stearate | 5g | - | 1g | - |
| | polyoxyl 20 cetyl ether | - | - | - | 0.125g |
| | PEG-400 | - | - | - | 0.25g |
| | Polysorbate 80 | - | 1g | - | - |
| Auxiliary solvent | ethanol | - | - | 0.1g | - |
| surfactant | Poloxamer 407 | 0.04g | - | - | - |
| | Poloxamer 188 | - | - | - | 0.25g |
| stabilizer | propylene glycol | 1.0g | 2g | - | - |
| | butylated hydroxytoluene | - | - | 0.0001g | - |
| thickener | Povidone K90 | 1.14g | | | |
| | hydroxypropyl methylcellulose | | | 0.3g | |
| buffer | Na₂HPO₄.H₂O | - | 0.42g | | 2.72g |
| | NaH₂PO₄.H₂O | - | 0.14g | 0.2g | 0.84g |
| | edetate sodium | | | 0.1 | |
| | citric acid | 0.016g | | | |
| | sodium citrate | 1.260g | | | |
| isotonic agent | NaCl | q.s. | 0.14g | 0.73g | |
| medium | purified water | q.s. | q.s. | q.s. | q.s. |
| stress test 40°C heating and shaking | initial content | 102.1% | 99.4% | 100.6% | 99.8% |
| | 10th day content | 102.0% | 87.3% not suitable | 84.9% not suitable | 35.1% not suitable |
| | 30th day content | 102.5% | 7.1% | - | - |
| | final properties | colorless and transparent | precipitation found | precipitation found | precipitation found |

### <Experimental example 4> Eye irritation test

An eye irritation test using rabbits was conducted as to composition 4-C obtained according to the present invention. Male New Zealand white rabbits were assigned into a washed group and not-washed group, and 0.1 mL of the test substance was applied per the applied site according to the Draize method. Evaluation was made according to the ocular lesion grade as in Table 9 below. The strength of eye irritation was sorted and evaluated according to the eye irritation table by Mean Index of Ocular Irritation (M.I.O.I), which is an average value dividing the sum of total points of each rabbit (I.I>o.I., The individual Index of Ocular Irritation, 1-110 points) by the number of rabbits at the determination date, the index of Acute Ocular irritation (I.A.O.A) which is a maximum value of the M.I.O.I during the observation period, and Individual Ocular Irritation Index of the 7^{th} day (I.O.I: points of each rabbit at 7th day), etc.

**[Table 9]**

| Evaluation | Evaluation values | | |
|---|---|---|---|
| | I.A.O.I | M.I.O.I | Day-7 I.O.I |
| None irritating | 0-5 | 0 (after 48 hours) | |
| Mild irritating | 5-15 | ≤5 (after 48 hours) | |
| Moderately irritating | 15-30 | ≤5 (after 4 days) | |
| Severe irritating | 30-60 | ≤20 (after 7 days) | ≤30 (all of 6 rabbits) |
| | | | ≤10 (at least 4 rabbits among 6 rabbits) |
| Extremely irritating | 60-80 | ≤40 (after 7 days) | ≤60 (all of 6 |
| | | | rabbits) ≤30 (at least 4 rabbits among 6 rabbits) |
| Maximally irritating | 80-110 | | |

As a result of evaluating the eye irritation as above, no dead animals, no general symptoms and no change in body weight in relation to the test substance was found during all testing periods. As a result of the ophthalmic test after applying the test substance, the I.A.O.I of the washed group and not-washed group was 0, which means no-irritation. Therefore, the present composition was evaluated not to be irritant.

### Industrial applicability

According to the present invention, a safe ophthalmic composition comprising cyclosporin with no excipient harmful to the human body may be easily prepared. In particular, the present invention comprises only ingredients whose safety has been confirmed and is a safe composition with no irritation. Thus, the composition of the present invention is more excellent than the conventional composition. Also, the composition has excellent safety, and is easily to prepared, and thus is advantageous in terms of saving cost.

## Claims

1. An ophthalmic composition comprising, in an aqueous-medium: cyclosporin as an active ingredient; and a polyoxyethylene stearate as a solubilizing agent.

2. The ophthalmic composition of claim 1, wherein the solubilizing agent is polyoxyl 40 stearate.

3. The ophthalmic composition of claim 2, further comprising a poloxamer.

4. The ophthalmic composition of claim 3, further comprising polyvinylpyrrolidone and propylene glycol.

5. The ophthalmic composition of claim 4, wherein poloxamer 407 is used as the poloxamer, and povidone K90 is used as the polyvinylpyrrolidone.

6. The ophthalmic composition of any one of claims 2 to 4, further comprising a buffer.

7. The ophthalmic composition of claim 6, wherein the buffer is at least one selected from a group consisting of citric acid, sodium citrate, edetate sodium, and aminocaproic acid.

8. The ophthalmic composition of claim 6, further comprising an isotonic agent.

9. The ophthalmic composition of claim 4, wherein the content of polyoxyl 40 stearate is 3-7 % by weight, the content of poloxamer is 0.01-0.04 % by weight, the content of polyvinylpyrrolidone is 0.01-1.8 % by weight, and the content of propylene glycol is 0.5-1.5 % by weight.

10. The ophthalmic composition of claim 9, wherein the content of cyclosporin is 0.05-0.1 % by weight.

11. A method of preparing an ophthalmic composition comprising cyclosporin, comprising:
(a) dissolving cyclosporin in polyoxyethylene stearate to prepare a first solution;
(b) dissolving poloxamer 407, propylene glycol, a thickener, an isotonic agent, and a buffer in water to prepare a second solution; and
(c) adding the second solution to the first solution and stirring the mixture.

12. The method of claim 11, wherein a homogenizer or a high-speed shear machine is not used in step (c).
